# EUROPEAN PATENT APPLICATION

(11) **EP 4 183 403 A1**
(43) Date of publication of application: **24.05.2023**
(21) Application number: 21209308.2
(22) Date of filing: 19.11.2021
(51) Int. Cl.: A61K 35/74, A61K 9/00, A61K 9/48, A61P 1/00, A61P 3/04, A61P 31/04

(54) **MODIFICATION OF FECAL MICROBIOTA WITH CAPSULES CONTAINING GRAM-POSITIVE BACTERIA**

(71) Applicant: Lietuvos sveikatos mokslu universitetas, 44307 Kaunas (LT)
(72) Inventor: Juozas, Kupcinskas, 44307 Kaunas (LT); Jurgita, Skieceviciene, 44307 Kaunas (LT); Jurga, Bernatoniene, 44307 Kaunas (LT); Vytautas, Kiudelis, 44307 Kaunas (LT); Ugné, Kulokiene, 44307 Kaunas (LT); Ruta, Inciuraite, 44307 Kaunas (LT)
(74) Representative: Zaboliene, Reda

(57) **Abstract**

We provide a method for preparing an oral capsule containing Gram-positive bacteria-enriched fecal-origin bacteria population. Bacteria were separated from the fecal matter by centrifugation and Gram-positive bacteria were further enriched by fluorescence activated cell sorting (FACS) method, using Gram-positive bacteria-specific (Wheat germ agglutinin, WGA) and/or DNA-specific (DAPI) markers. Capsules containing Gram-positive bacteria-enriched bacterial population may benefit for a higher effectiveness of fecal microbiota transplantation in inflammatory bowel disease patients or in patients with other conditions where a higher number of Gram-positive bacteria is required. These conditions, that are associated with low-grade gut inflammation mediated by certain Gram-positive bacteria, include obesity with metabolic syndrome, diabetes mellitus, and even some variants of irritable bowel syndrome. We also provide results of the bacterial composition analysis that was performed on the constituents of the capsule, that revealed a significant increase of Gram-positive bacteria in the sorted bacterial samples with the highest number of Gram-positive bacteria in samples sorted based on the WGA marker.

## Description

### FIELD OF THE INVENTION

This invention relates to the use of Gram-positive bacteria for modification of human fecal microbiota. Gram-positive bacteria are separated from donor fecal matter and placed in capsules that carry them to the digestive tract of the recipient.

### DESCRIPTION OF THE RELATED ART

In recent years, a great deal of progress has been made in understanding the role of human fecal microbiota in maintaining a healthy host homeostasis and in the pathogenesis of various diseases. Therefore, fecal microbiota modification is being intensively investigated as a possible treatment option for various diseases and pathological conditions (e. g. inflammatory bowel diseases, cardiovascular diseases, obesity and metabolic syndrome), or as a way to modify response to therapy (e. g. checkpoint inhibitors).

Currently, fecal microbiota transplantation is the most effective method of treating recurrent *C*/*ostridioides difficile* colitis and the only reliable option of microbiota modification that is used in clinical practice. However, fecal microbiota transplantation does not show such consistent results when used to treat other conditions, e. g. inflammatory bowel diseases (Narula, 2017). The reason for these differences in effectiveness is that *Clostridioides difficile* colitis is treated by an overall increase in variety of healthy gut microflora while inflammatory bowel diseases are linked with dysregulation of specific bacteria and their metabolites, thus increasing the gut microbiota count is not always sufficient to correct this imbalance and the effect is heavily dependent on the donor's microbiota composition. Studies have shown that inflammatory bowel disease patients have a higher chance of achieving remission after fecal microbiota transplantation when receiving microbiota rich in specific Gram-positive bacteria (e. g. *Eubacterium, Roseburia, Ruminococcus*), while certain Gram-negative bacteria (e. g. *Fusobacterium, Sutterella, Prevotella*) are linked to treatment failure (Paramsothy, 2019).

US2021236562 discloses methods and compositions for the treatment of skin conditions associated with dysbiosis. Compositions include single or more than one strain of healthy donor derived bacteria for administration to provide therapy for skin conditions associated with dysregulated microbiota. Such compositions include gram-negative and/or gram-positive bacteria.

Therefore, a more precise method of fecal microbiota modification is needed, which provides the highest number of beneficial bacteria while containing as few harmful bacteria as possible. By separating Gram-positive bacteria from donor fecal matter and delivering them using oral capsules, which have been proven to be reliable in fecal microbiota transplantation (Kao, 2017), it is reasonable to expect higher effectiveness of fecal microbiota transplantation in inflammatory bowel disease patients or in patients with other conditions where a higher number of Gram-positive bacteria is required.

### SUMMARY OF THE INVENTION

This invention describes an oral capsule consisting of donor fecal microbiota enriched with Gram-positive bacteria. Based on the studies on inflammatory bowel disease (IBD) patients, higher numbers of Gram-positive bacteria and lower numbers of harmful Gram-negative bacteria in a donor fecal matter are expected to bring more favourable outcomes of fecal microbiota transplantation. This may also be useful in other conditions associated with depletion of key Gram-positive bacteria, such as obesity with metabolic syndrome, diabetes mellitus, irritable bowel syndrome, constipation and traveller's diarrhoea.

A method of the preparation of the Gram-positive bacteria-enriched capsule containing Gram-positive bacteria, comprises the steps of:
(a) preparing the donor fecal matter;
(b) providing the bacteria sample by isolation of bacteria from the fecal matter;
(c) bacterial Gram staining and fluorescence activated cell sorting;
(d) preparing a powder of Gram-positive bacteria and carrier;
(e) encapsulating the powder into single-layered capsules with enteric coating.

The isolation of fecal bacteria from the fecal matter according to step (b) is performed by centrifugation and washing steps.

The bacterial gram staining according to step (c) is performed using fluorescently labelled WGA - WGA+ or fluorescently labelled WGA and DAPI - WGA+/DAPI+ dyes. According to the best mode the bacterial gram staining is performed using fluorescently labelled WGA - WGA+ dye. The fluorescence activated sorting of bacteria from fecal matter is based on the expression of two fluorescently labelled WGA and DAPI - WGA+/DAPI+ or fluorescently labelled WGA- WGA+. According to the best mode the fluorescence activated sorting of bacteria from fecal matter is based on WGA signal intensity.

The Gram-positive bacteria-enriched bacterial samples are prepared for encapsulation in powder form and after the powder is encapsulated in single-layered capsules with enteric coating to avoid release in the stomach. The capsule is prepared for oral administration. The at least one species of Gram-positive bacteria is present in an amount sufficient for a reduction in Staphylococcus aureus in the subject. The samples contained 4-5 times more reads belong to gram-positive bacteria than Gram-negative bacteria.

According to another embodiment invention provides the Gram-positive bacteria-enriched capsules made according to the method described.

The capsules is intended for use in the treatment of the pathological conditions related with intestine tract, the obesity with metabolic syndrome, diabetes mellitus, irritable bowel syndrome, constipation and traveller's diarrhoea, especially for the treatment of the inflammatory bowel disease in patients.

### BRIEF DESCRIPTION OF DRAWINGS

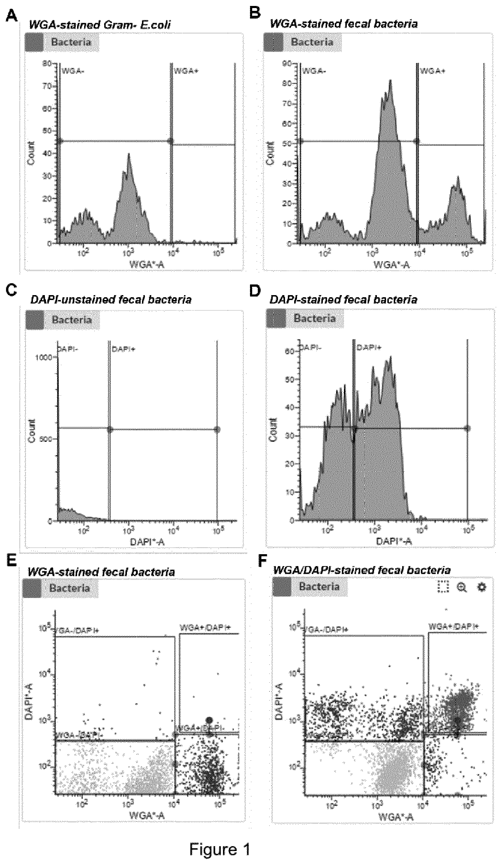

Figure 1 illustrates gating strategy for the fluorescence activated sorting of Gram positive (Gram+) bacteria. A - WGA-stained Gram negative (Gram-) E.coli bacteria population was analysed to gate the WGA- (Gram-) cells. B - WGA-stained Fecal bacteria populations: Gram- (WGA-) and Gram+ (WGA+). C-D - DAPI negative (DAPI-) and DAPI positive (DAPI+) populations gated to select only cells containing DNA. E - WGA+/DAPI-gate (lower right corner) selected for sorting of Gram+ bacteria in the case of WGA staining. F - WGA+/DAPI+ gate (upper right corner) selected for sorting of Gram+ bacteria in the case of WGA and DAPI staining.
Figure 2 is overview of raw bacterial sequences (ASVs) in three different samples (Sorted WGA+ and WGA+/DAPI+ and unsorted bacteria sample). A-B - Venn diagram of common and unique bacterial sequences. C - table of detected ASVs and sequencing reads. D - comparison table of study samples.
Figure 3 illustrates microbiota composition comparison. A - multidimensional scaling (MDS) plot. B-E -comparison of the bacterial alpha diversity based on the bacterial sequence profiles.
Figure 4 is comparison of most abundant bacterial taxonomic groups between three different samples. A- comparison of Gram-positive bacterial groups. B- comparison of Gram-negative bacterial groups.

### DETAILED DESCRIPTION OF THE INVENTION

### Preparation of the donor fecal matter

The donor fecal matter is produced from stool that is donated by healthy volunteers. Before becoming donors, the volunteers have to sign an informed consent form and undergo the selection process. The process starts from a medical interview, which aims to assess the donor's risk of infections and past interventions that might have altered the gut microbiota. After that, blood and stool tests for potentially transmissible infectious diseases are performed. After passing the selection process, the volunteer may start donating stool, which is processed according to a standardized protocol, frozen in -80°C and stored until use. The entire donor selection and testing process, the processing and banking of the stool samples is performed according to international recommendations (Cammarota, 2019).

### Isolation of bacteria from the fecal matter

The frozen fecal matter was thawed overnight at 4°C and centrifuged at 200 × g in a 50 mL centrifuge tube for 10 minutes to sediment larger pieces. Supernatant was collected and transferred to a new 1.5 mL microcentrifuge tube and centrifuged at 10 000 × g for 8 minutes to pellet the fecal bacteria. The supernatant was discarded, bacteria were washed by resuspending pellet in 1 mL 0.22 µm sterile filtered phosphate buffered saline (PBS) and transferred to a new 1.5 mL tube. The centrifugation and washing steps were repeated 5-7 times until the supernatant appeared clear. After the final centrifugation, the pellet was resuspended in 1 mL of sodium chloride solution containing 0.25% bovine serum albumin.

### Bacterial Gram staining and fluorescence activated cell sorting (FACS)

Prepared bacteria samples were used to distinguish between Gram-positive (Gram+) and Gram-negative (Gram-) bacteria based on the CF^{®}488A-conjugated Wheat Germ Agglutinin (WGA) (binding to the peptidoglycan layer of the Gram+ bacteria) signal using Bacterial Viability and Gram Stain Kit (Biotium, cat. No. #32001) and FACS Melody Cell sorter (BD Biosciences). DAPI stain was used as a counterstain labelling all the bacteria. Before proceeding to the gram staining, 20 µl of prepared bacteria sample was diluted in 980 µl of 0.22 µm sterile filtered PBS. 50 µl of diluted bacteria were transferred into a new 1.5 mL tube for staining and 2.5 µL WGA stain was added to the sample and incubated at room temperature for 10 min in the dark. Sample was centrifuged at 10 000 × g for 5 min and the supernatant containing WGA staining solution was discarded. The remaining pellet was resuspended in 50 µL of 0.22 µm sterile filtered PBS, 1 µL of DAPI stain was added to the sample tube and incubated at room temperature for 5 min in the dark. Samples were transferred into a new 5 ml cytometric tubes containing 200 µL of 0.22 µm sterile filtered PBS and were loaded to the FACS Melody sorter. Gram-positive bacteria were sorted based on WGA and DAPI signal intensities (WGA+/DAPI+ (in the case of WGA and DAPI staining) or WGA+ (in the case of WGA staining only)). WGA-stained Gram-negative *E.coli* bacteria population (SHuffle^{®} T7 Competent *E*. *coli,* New England BioLabs, UK) was used to set the WGA- gate as WGA does not bind to cell wall of the Gram-negative bacteria. Unstained bacteria isolated from the fecal matter was used to set the gate for the DAPI- events.

### Examination of bacterial composition

To study the bacterial composition of the sorted samples, a bacterial 16S rRNA gene analysis was performed. DNA was extracted from both unsorted and sorted bacteria populations using QIAamp Fast DNA Stool Mini Kit (Qiagen, Ref. 51604) according to protocol "Isolation of DNA from Stool for Pathogen Detection" with slight modifications (initial volume of 500 µL of sample was mixed with 1 mL of Inhibit EX Buffer at the beginning of the protocol; the suspension was heated at 95°C for 5 min to better lyse the Gram-positive bacteria; 100 µl of Buffer ATE was used for elution of the DNA). The concentration of DNA was evaluated using NanoDrop 2000 spectrophotometer.

The 16S rRNA gene library preparation and sequencing were performed as previously described (Wang, 2016). Briefly, 16S rRNA V1-V2 region-specific set of primers, where primers are 27F, SEQ ID No: (5'-AGAGTTTGATCCTGGCTCAG-3') and 338R, SEQ ID No: (5'-TGCTGCCTCCCGTAGGAGT-3') (HPLC purified DNA custom oligos, Metabion, GenBank with accession number FJ961336) - was used in the process of PCR (cycling conditions: (98 °C, 30 s; 30 × [98 °C, 9 s; 55 °C, 60 s; 72 °C, 90 s]; 72 °C, 10 min; 10 °C, infinity) (Kozich, 2013). For the purification of PCR products and normalization of the amplicons, the Invitrogen SequalPrep Normalization Plate Kit was used (Thermo Fisher Scientific, Waltham, USA). 16S rRNA gene sequencing was performed on the Illumina MiSeq platform using MiSeq Reagent Kit v3 in accordance with the manufacturer's instructions.

Bioinformatic processing and statistical analysis were performed as previously described (Camarinha-Silva, 2014). FastQ files were aligned with SILVA database (v138) using R (v4.0.4) (R Core Team, 2020) package dada2 (Callahan, 2016) v1 .10.1. Bacterial sequences represented by Amplicon Sequence Variants (ASVs) were annotated to a taxonomic affiliation based on the naïve Bayesian classification (Wang, 2007) with a pseudo-bootstrap threshold of 80%. ASVs that were found in low numbers (ASVs found only in one of the samples with the number of reads < 35, as well as ASVs found in two samples with the number of reads < 50) were excluded from the analysis as possible sequencing errors. Relative abundances (expressed as percentages) of different microbial communities' phylogenetic ranks (phylum, class, order, family, genus) were used to examine bacterial composition of studied samples. Bacterial richness and alpha-diversity indices (Shannon, Simpson, Pielou evenness) were calculated using R package vegan v 2.5-7 (Oksanen, 2015). The data matrices comprising the percentage of abundances of each of the above-mentioned taxa were used to construct sample-similarity matrices using the Euclidean distance, where samples were ordinated by multidimensional scaling (MDS) using R.

### Encapsulation of bacteria

Gram-positive bacteria-enriched bacterial samples are prepared for encapsulation using Liquisolid technology (Spireas, 2002) which is adapted to powder a liquid consisting of bacteria collected in a PBS solution. The samples are prepared as follows: 15-30 g, preferably 25.0 g of carrier, which is selected from magnesium aluminium metasilicate, anhydrous colloidal silicon dioxide (Aerosil), carbomers, carrageenan, and 5-20 g, preferably 10.0 g of the bacterial solution are mixed in a mortar for production, sieved through a 1 mm sieve and homogenized in a mixer for 10 min. The prepared mixture is placed in a dyer at 60 °C for 1 hour, and the procedure is repeated until all the bacterial solution has been used up. The prepared mixture is placed in a ventilated dryer at 40 °C for 24 hours. The powder is then placed in single-layered capsules with enteric coating to avoid release in the stomach.

### Results

Gram-positive bacteria were sorted based on the expression of fluorescently labelled WGA that binds specifically to the N-acetylglucosamine of the peptidoglycan layer of Gram-positive bacteria. Samples stained with one (fluorescently labelled WGA - WGA+) or two (fluorescently labelled WGA and DAPI - WGA+/DAPI+) dyes were prepared for sorting. In total, 139 495 WGA+ cells were collected from the WGA-stained sample (Fig 1. (E)) and 115 184 WGA+/DAPI+ - from the WGA/DAPI-stained sample (Fig 1. (F)). After the DNA extraction, DNA concentrations measured were 1.2 ng/µL, 3.3 ng/µL and 2.7 ng/µL in WGA+, WGA+/DAPI+ and unsorted sample, respectively.

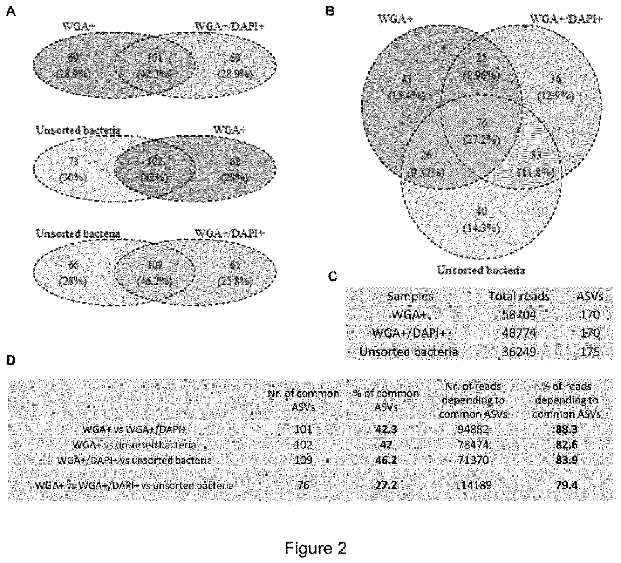

16S RNA gene sequencing analysis revealed that sorted WGA+ and WGA+/DAPI+ bacteria samples shared 42.3% of the total ASVs (Fig. 2 A). A similar percentage of common ASVs were found when sorted bacteria samples were compared with the unsorted bacteria sample (Fig. 2 A). Common ASVs for all three samples amounted 27.2% (Fig. 2 B). It is noteworthy that reads belonging to common ASVs made up the majority of the total sequenced reads (-84%), indicating that common ASVs constitute a core of the bacterial community in the studied samples (Fig. 2 D).

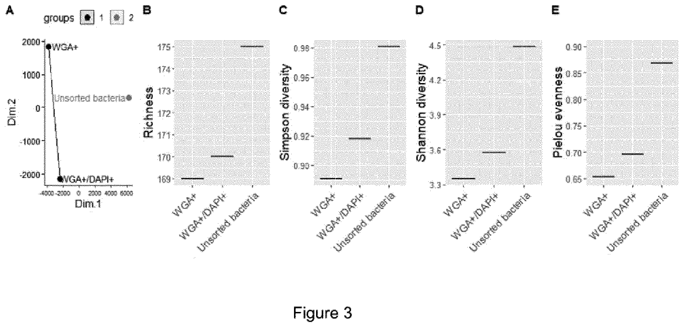

According to MDS analysis, sorted bacteria samples were more similar to each other than to the unsorted bacteria sample (Fig. 3 A). In the unsorted sample, which should theoretically contain both Gram-positive and Gram-negative bacteria, bacterial richness, as well as bacterial diversity (measured by Simpson and Shannon diversity indices) and bacterial evenness (measured by Pielou evenness index) were higher (Fig. 3 B-E). In the WGA+/DAPI+ sample bacterial alpha-diversity was slightly higher than in the WGA+ sample.

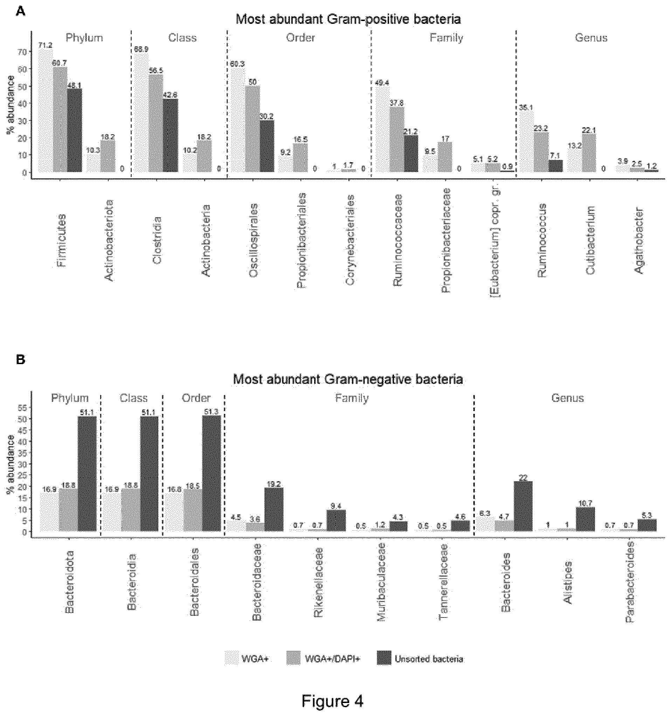

The bacterial composition studied at different taxonomic levels (phylum, class, order, family, and genus) revealed a significant increase in Gram-positive bacteria (Fig. 4 A) and a significant decrease in Gram-negative bacteria (Fig. 4 B) in the sorted bacteria samples. Moreover, in almost all groups of the most abundant bacteria, the percentage of Gram-positive bacteria is higher in the WGA+ sample than in the WGA+/DAPI+ sample. A similar tendency was observed for Gram-negative bacteria: Gram- bacteria were less abundant in the WGA+ sample than in the WGA+/DAPI+ sample.

Finally, the percentage of reads belonging to Gram-positive and Gram-negative bacteria in the unsorted sample were similar (~48% and ~52%, respectively) (Table 1), while sorted samples contained 4-5 times more reads belonging to Gram-positive bacteria than Gram-negative bacteria. In the WGA+ sample, an increased number of Gram-positive bacterial reads and a reduced number of Gram-negative bacterial reads was observed compared to the WGA+/DAPI+ sample.

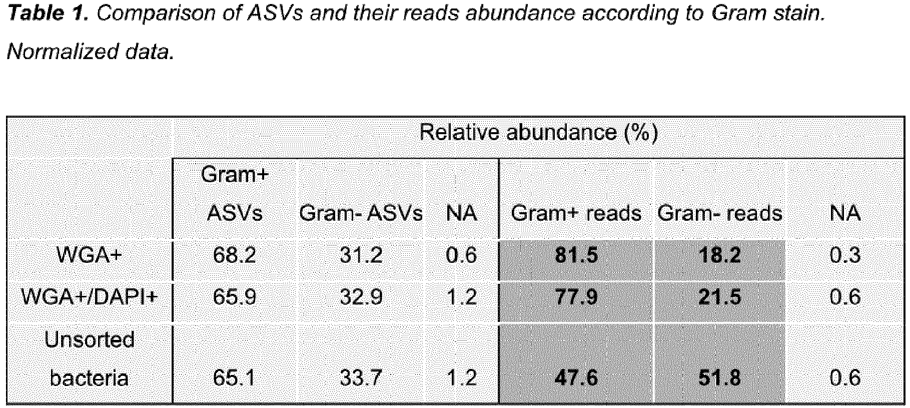

Fluorescence activated sorting of bacteria from fecal matter, using fluorescently labeled WGA or a combination of WGA and DAPI stains, increased the number of Gram-positive bacteria and decreased the number of Gram-negative bacteria in the sample compared to the unsorted sample.

Sorting based only on WGA labeling provided more Gram-positive and less Gram-negative bacteria in the final sample compared to the sorting using a combination of WGA and DAPI stain.

### REFERENCES

- Callahan BJ, Mcmurdie PJ, Rosen MJ, et al. DADA2: High-resolution sample inference from Illumina amplicon data. 2016;13(7).
- Camarinha-Silva A, Jáuregui R, Chaves-Moreno D, et al. Comparing the anterior nare bacterial community of two discrete human populations using Illumina amplicon sequencing. Environ Microbiol. 2014;16(9):2939-52.
- Cammarota G, laniro G, Kelly CR, et al. International consensus conference on stool banking for faecal microbiota transplantation in clinical practice. Gut. 2019;68(12):2111-21.
- Kao D, Roach B, Silva M, et al. Effect of oral capsule- vs colonoscopy-delivered fecal microbiota transplantation on recurrent Clostridium difficile infection: A randomized clinical trial. JAMA - J. Am. Med. Assoc. 2017;318:1985-1993.
- Kozich JJ, Westcott SL, Baxter NT, et al. Development of a dual-index sequencing strategy and curation pipeline for analyzing amplicon sequence data on the MiSeq Illumina sequencing platform. Appl Environ Microbiol. 2013;79(17):5112-20.
- Narula N, Kassam Z, Yuan Y, et al. Systematic Review and Meta-analysis: Fecal Microbiota Transplantation for Treatment of Active Ulcerative Colitis. Inflamm. Bowel Dis. 2017;23:1702-1709.
- Oksanen AJ, Blanchet FG, Friendly M, et al. Vegan: Community Ecology Package. Package 'vegan.' 2015. Available at: https://CRAN.R-project.org/package=vegan.
- Paramsothy S, Nielsen S, Kamm MA, et al. Specific Bacteria and Metabolites Associated With Response to Fecal Microbiota Transplantation in Patients With Ulcerative Colitis. Gastroenterology. 2019;156:1440-1454.e2.
- R Core Team (2020). R: A language and environment for statistical computing. R Foundation for Statistical Computing, Vienna, Austria. Available at: https://www.R-project.org/.
- Spireas S. Liquisolid system and method of preparing same. U.S Patent; 6423339B1 (2002).
- Wang J, Thingholm LB, Skiecevič̌iene J, et al. Genome-wide association analysis identifies variation in Vitamin D receptor and other host factors influencing the gut microbiota. Nat Genet. 2016;48(11): 1396-406.
- Wang Q, Garrity GM, Tiedje JM, et al. Naive Bayesian Classifier for Rapid Assignment of rRNA Sequences into the New Bacterial Taxonomy. Appl Environ Microbiol. 2007;73(16):5261-7.

Organization Applicant
----------------------

<110> OrganizationName : Lietuvos sveikatos moksl universitetas

Application Project
-------------------
<120> Title : MODIFICATION OF FECAL MICROBIOTA WITH CAPSULES CONTAINING
GRAM-POSITIVE BACTERIA
<130> AppFileReference : EPP2021/
<141> CurrentFilingDate : 2021-11-19

Sequence
<212> Type: DNA
<211> Lenght: 20
--------
<213> OrganismName : bacteria
<221> FeatureKey: primer
<400> SEQ ID NO:1

agagtttgat cctggctcag                                                20

<212> Type: DNA
<211> Lenght: 19
--------
<213> OrganismName : bacteria
<221> FeatureKey: primer
<400> SEQ ID NO:2

tgctgcctcc cgtaggagt                                                 19

## Claims

1. A method of the preparation of the Gram-positive bacteria-enriched capsule containing Gram-positive bacteria, wherein the method that comprising the steps of:
(a) preparing the donor fecal matter;
(b) providing the bacteria sample by isolation of bacteria from the fecal matter;
(c) bacterial gram staining and fluorescence activated cell sorting;
(d) preparing a powder of Gram-positive bacteria and carrier;
(e) encapsulating the powder into single-layered capsules with enteric coating.

2. The method of the preparation of the Gram-positive bacteria-enriched capsules according to claim 1, wherein isolation of fecal bacteria from the fecal matter is performed by centrifugation and washing steps.

3. The method of the preparation of the Gram-positive bacteria-enriched capsules according to claims 1 or 2, wherein bacterial gram staining is performed using fluorescently labelled WGA - WGA+ or fluorescently labelled WGA and DAPI - WGA+/DAPI+ dyes.

4. The method of the preparation of the Gram-positive bacteria-enriched capsules according to claim 3, wherein bacterial gram staining is performed using fluorescently labelled WGA - WGA+ dye.

5. The method of the preparation of the Gram-positive bacteria-enriched capsules according to any of claims 1-4, wherein fluorescence activated sorting of bacteria from fecal matter is based on the expression of two fluorescently labelled WGA and DAPI - WGA+/DAPI+ or fluorescently labelled WGA- WGA+.

6. The method of the preparation of the Gram-positive bacteria-enriched capsules according to claim 5, wherein fluorescence activated sorting of bacteria from fecal matter is based on WGA signal intensity.

7. The method of the preparation of the Gram-positive bacteria-enriched capsules according to any of claims 1-6, wherein Gram-positive bacteria-enriched bacterial samples are prepared for encapsulation in powder form.

8. The method of the preparation of the Gram-positive bacteria-enriched capsules according to any of claims 1-7, wherein the powder is encapsulated in single-layered capsules with enteric coating to avoid release in the stomach.

9. The method of the preparation of the Gram-positive bacteria-enriched capsules according to any of claims 1-8, wherein the capsule is prepared for oral administration.

10. The method of the preparation of the Gram-positive bacteria-enriched capsules according to any of claims 1-9, wherein the at least one species of Gram-positive bacteria is present in an amount sufficient for a reduction in Staphylococcus aureus in the subject.

11. The method of the preparation of the Gram-positive bacteria-enriched capsules according to any of claims 1-10, wherein samples contained 4-5 times more reads belonging to Gram-positive bacteria than Gram-negative bacteria.

12. A Gram-positive bacteria-enriched capsules made according to the process of any of claims 1-11.

13. Gram-positive bacteria-enriched capsules according to claim 12 for use in the treatment of the pathological conditions related with intestine tract, the obesity with metabolic syndrome, diabetes mellitus, irritable bowel syndrome, constipation and traveller's diarrhoea.

14. Gram-positive bacteria-enriched capsules according to claim 13 for use in the treatment of the inflammatory bowel disease in patients.
